(19) **Europäisches Patentamt**

**European Patent Office**

**Office européen des brevets**

(11) **EP 0 974 304 A1**

(12) **EUROPÄISCHE PATENTANMELDUNG**

(43) Veröffentlichungstag:
26.01.2000 Patentblatt 2000/04

(51) Int. Cl.[7]: **A61B 5/11**, A61B 5/048

(21) Anmeldenummer: **98122563.4**

(22) Anmeldetag: **02.12.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **24.07.1998 DE 19833497**

(71) Anmelder:
**Dimpfel, Wilfried, Prof. Dr.**
**35440 Linden (DE)**

(72) Erfinder:
**Dimpfel, Wilfried, Prof. Dr.**
**35440 Linden (DE)**

(74) Vertreter: **HOFFMANN - EITLE**
**Patent- und Rechtsanwälte**
**Arabellastrasse 4**
**81925 München (DE)**

(54) **Vorrichtung zur Bestimmung der Schlaftiefe**

(57)     Die Erfindung betrifft eine Vorrichtung zur Bestimmung der Schlaftiefe des Menschen und umfaßt eine Transformationseinrichtung, die ein EEG-Signal in den Frequenzbereich transformiert, und eine Verarbeitungseinrichtung, die zumindest zwei von drei Frequenzbereichen für die Ermittlung eines Schlaftiefe-Index $S_F$ verwendet.

**Fig. 1**

EP 0 974 304 A1

**Beschreibung**

Die vorliegende Erfindung betrifft eine Vorrichtung zur Bestimmung der Schlaftiefe, insbesondere während der Narkose bei einem therapeutischen/chirurgischen Eingriff.

Die Bestimmung der Schlaftiefe sowohl bei physiologischem als auch bei medikamenten-induziertem Schlaf erfolgt in der Regel visuell durch erfahrene Experten, die das EEG eines Patienten auswerten und einzelnen 20 bis 30 Sekunden langen Zeitabschnitten Werte zuweisen, die die Schlaftiefe wiedergeben. Die Bewertung basiert auf Arbeiten von Rechtschaffen & Kales aus dem Jahre 1969 und ist heutzutage der einzige allgemein anerkannte Standard für die Beurteilung und Ermittlung der Schlaftiefe des Menschen auf der Grundlage eines EEG.

Es ist offensichtlich, daß sich dieses Verfahren nicht dafür eignet, in größerem Maßstab oder während eines therapeutischen/chirurgischen Eingriffs durchgeführt zu werden, für den der Patient in einen medikamenten-induzierten Schlaf (Narkose) versetzt wird. Aus diesem Grund sind zahlreiche Versuche unternommen worden, die Schlaftiefe auf der Basis des EEG-Signals zu bestimmen. Insbesondere wurden dazu EEG-Signale transformiert (Fast-Fourrier-Transformation) und die Darstellung des EEG-Signals im Frequenzbereich einer Auswertung unterzogen. Besondere Berücksichtigung fanden dabei Frequenzen, die allgemein als $\Delta$-Wellen bezeichnet wurden, und die in einem Bereich von etwa 0 bis 3,5 Hz lagen. Diese sehr langwelligen Anteile des EEG-Signals wurden mit dem Bewußtseinszustand "Schlaf" in Verbindung gebracht und Methoden entwickelt, die auf der Grundlage dieser Frequenzen eine Bestimmung der Schlaftiefe ermöglichen sollten. Die Ergebnisse wurden dadurch nachgeprüft, daß die automatisch bestimmte Schlaftiefe verglichen wurde mit der von Experten visuell bestimmten Schlaftiefe nach dem anerkannten Verfahren von Rechtschaffen & Kales. Die bisher eingesetzten Methoden führten aber zu keinen verläßlichen oder nur sehr eingeschränkt verwendbaren Ergebnissen, so daß letztlich eine zuverlässige vollautomatische Bestimmung der Schlaftiefe bis heute nicht möglich ist.

Neben den zuvor beschriebenen Bemühungen, den Bewußtseinszustand "Schlaf" allgemeingültig auf automatisierbare Weise zu bestimmen, gab es Versuche, insbesondere medikamenten-induzierten Schlaf zu bewerten. Dabei wurden teilweise einzelne Frequenzbereiche auch außerhalb des Bereichs der $\Delta$-Wellen untersucht. Auch dabei war der Erfolg nur mäßig und überdies auf das jeweils untersuchte Medikament beschränkt, so daß die Ergebnisse nicht auf andere Medikamente übertragen werden konnten. Auch die Validierung der Ergebnisse über eine visuelle Bewertung auf der Basis von Rechtschaffen & Kales zeigte Defizite, so daß von einer zuverlässigen Bewertung des menschlichen Schlafes kaum gesprochen werden kann.

Nicht nur für wissenschaftliche oder medizinische Untersuchungen und Anwendungen ist die Kenntnis der Schlaftiefe von Interesse. Das Ausbleiben des medikamenten-induzierten Schlafes während eines therapeutischen/chirurgischen Eingriffes stellt eine für den Nicht-betroffenen kaum vorstellbare Belastung des Patienten dar. Dies gilt umso mehr, wenn neben dem Ausbleiben des medikamenten-induzierten Schlafes auch die medikamenten-induzierte Schmerzunempfindlichkeit nicht erzielt wird und gleichzeitig, die bei Narkosen regelmäßig vorgesehene Paralysierung der Muskelaktivitäten erfolgreich induziert wird. Für die Patienten bedeutet dies letztlich, daß ein therapeutischer/chirurgischer Eingriff vorgenommen wird, ohne daß der Patient schläft und schmerzunempfindlich ist, aber auch ohne daß er in der Lage ist, auf seinen Zustand aufmerksam zu machen. Durch eine zuverlässige Erkennung des Schlafes, die vollautomatisch durchgeführt wird und während eines therapeutischen/chirurgischen Eingriffs und der damit verbundenen Narkose mitläuft, kann hier Abhilfe geschafft werden.

Aber unabhängig von den zuvor geschilderten und üblicherweise seltenen Fall des Narkoseversagens besteht grundsätzliches Interesse an einem zuverlässigen Verfahren zur qualifizierten und quantitativen Bestimmung des Bewußtseinszustands Schlaf, so daß die der Erfindung zugrunde liegende Aufgabe darin zu sehen ist, eine Vorrichtung zu schaffen, mit der die Schlaftiefe zuverlässig und vollautomatisch auf der Basis des EEG-Signals bestimmt werden kann.

Gelöst wird diese Aufgabe durch eine Vorrichtung mit den Merkmalen des Patentanspruchs 1. Vorteilhafte Ausgestaltungen ergeben sich aus den Unteransprüchen.

Die Erfindung basiert auf der Erkenntnis, daß von drei charakteristischen Bereichen des im Frequenzbereich dargestellten EEG-Signals zumindest zwei herangezogen werden müssen, um einen Schlaftiefe-Index $S_F$ zu bestimmen. Entscheidend ist, daß das EEG-Signal in Bezug auf diese Bereiche untersucht wird und unter Berücksichtigung von zumindest zwei, vorzugsweise aber aller drei Bereiche der Schlaftiefe.-Index $S_F$ ermittelt wird.

Im folgenden wird ein Ausführungsbeispiel der erfindungsgemäßen Vorrichtung unter Bezugnahme auf die Figuren genauer beschrieben, in denen zeigt:

Fig. 1    schematisch den Aufbau eines Ausführungsbeispiels der erfindungsgemäßen Vorrichtung;

Fig. 2    schematisch den Verlauf des EEG-Signals im Frequenzbereich sowie die erfindungsgemäß zu berücksichtigenden Bereiche.

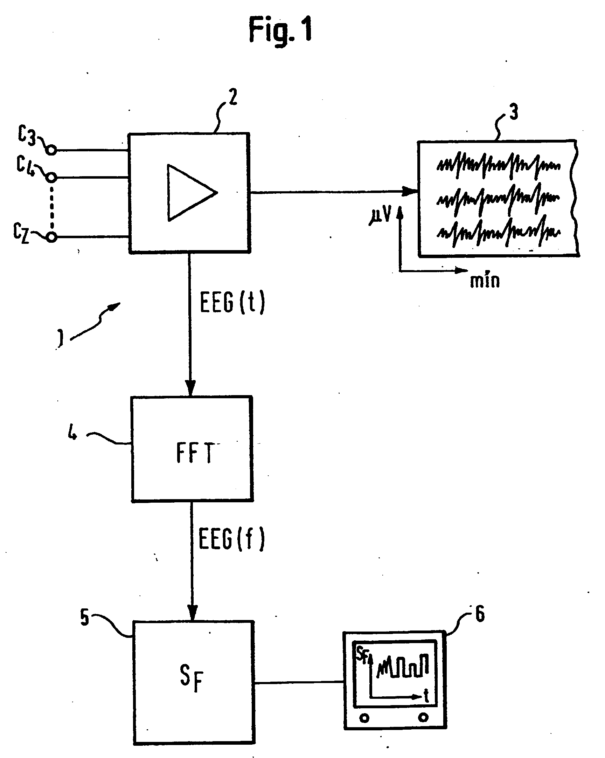

In Fig. 1 ist schematisch ein Ausführungsbeispiel der erfindungsgemäßen Vorrichtung dargestellt. Ein Ausführungsbeispiel umfaßt eine EEG-Meßapparatur 1, von der in Fig. 1 nur die Elektroden C3 und C4 sowie die Elektrode $C_Z$ dargestellt sind. Üblicherweise umfaßt eine Standard-EEG-Meßapparatur 15-25 Elektroden zusätzlich zu der Elektrode $C_Z$. Die Ausgangssignale der Elektroden werden einer Verstärkereinrichtung 2 zugeführt, die die Elektrodensignale so verstärkt und verarbeitet, daß damit ein Schreiber (nicht dargestellt) angesteuert werden kann, der das EEG 3 schreibt. Bei der erfindungsgemäßen Vorrichtung wird ein EEG-Signal, vorzugsweise das der Elektroden $C_3$ und/oder $C_4$, aus der EEG-Meßapparatur 2 abgeleitet und einer Transformationseinrichtung 4 zur Durchführung einer Fourriertransformation (Fast Fourrier Transformation, FFT) zugeführt. Die Verbindung zwischen der EEG-Meßapparatur 2 und der Transformationseinrichtung 4 kann auf verschiedene Weise hergestellt werden, vorzugsweise über eine optische Verbindung, beispielsweise eine Glasfaser, so daß auch bei größeren Übertragungsstrecken eine Störung des übertragenen EEG-Signals sicher ausgeschlossen wird.

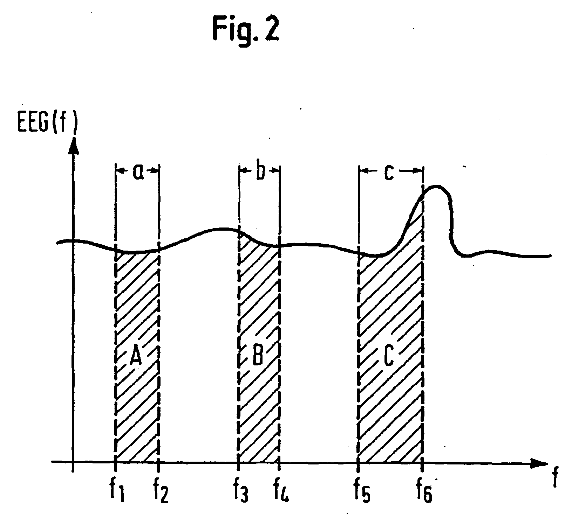

Das Ausgangssignal der Transformationseinrichtung 4 gibt das Fourrier-transformiete EEG-Signal an eine Verarbeitungseinrichtung 5 weiter, die das nun im Frequenzbereich vorliegende EEG-Signal EEG(f) weiter verarbeitet. Dazu werden drei Frequenzbereiche des transformierten EEG-Signals genauer untersucht. Die Frequenzbereiche sind in Fig. 2 dargestellt, wobei der Frequenzbereich a von 4,75 bis 6,75 Hz, der Frequenzbereich b von 12,75 bis 18,5 Hz und der Frequenzbereich c von 18,75 bis 35,0 Hz reicht. Die Verarbeitungseinrichtung 5 bestimmt für jeden der Frequenzbereiche einen repräsentativen Wert A, B und C und ermittelt daraus den die Schlaftiefe kennzeichnenden Index

$$S_F = \frac{A + B}{C}.$$

Der Schlaftiefe-Index $S_F$ reflektiert die Schlaftiefe des Patienten zuverlässig, wie in einer Validierungsstudie gegenüber den nach Rechtschaffen & Kales ermittelten Schlafzuständen nachgewiesen werden konnte.

Die für die drei Frequenzbereiche a, b und c repräsentativen Werte A, B und C können auf verschiedene Weise gewonnen werden. Entscheidend ist, daß zumindest zwei der Frequenzbereiche a, b und c, vorzugsweise aber alle drei, vollständig oder teilweise bei der Bestimmung der repräsentativen Werte berücksichtigt werden. Ein Verfahren zur Bestimmung repräsentativer Werte besteht in der Integration des transformierten EEG-Signals von der Anfangsfrequenz bis zur Endfrequenz des jeweiligen Bereichs bzw. eines Ausschnitts innerhalb des Bereichs.

In Fig. 2 geben die schraffierten Flächen die repräsentativen Werte A, B und C wieder, die der Integration des transformierten EEG Signals in den Frequenzbereichen a, b und c ermittelt werden. Als repräsentativer Wert eignet sich grundsätzlich aber auch ein Mittelwert des transformierten EEG-Signals, der über die einzelnen Frequenzbereiche a, b und c bestimmt wird.

Wie in Fig. 1 gezeigt, umfaßt das hier beschriebene Ausführungsbeispiel der erfindungsgemäßen Vorrichtung einen Monitor 6 zur Darstellung des Schlaftiefe-Index $S_F$ Neben einer Darstellung auf einem Monitor kann das Signal $S_F$ auch für andere Zwecke, beispielsweise eine Alarmierung, genutzt werden. Das Signal $S_F$ kann auch dem (nicht dargestellten) EEG-Schreiber zugeführt werden, so daß auf dem EEG 3 auch der Schlaftiefe-Index $S_F$ wiedergegeben wird.

Zu beachten ist, daß die erfindungsgemäße Vorrichtung einen Index angibt, der in dieser Form bislang nicht bestimmt wurde und der als Schlaftiefe-Index $S_F$ bezeichnet wird. Dieser Index unterscheidet sich von den bisher ermittelten Werten und gestattet die Bestimmung physiologischen und medikamenten-induzierten Schlafs, unabhängig vom eingesetzten Medikament.

Die Transformationseinrichtung und die Verarbeitungseinrichtung sind vorzugsweise in einem Personal Computer zusammengefaßt, der auch die Rechenleistung zur Verfügung stellen kann, die für die Transformation des EEG-Signals in den Frequenzbereich und/oder die Verarbeitung des transformierten EEG-Signals, d.h. die Bestimmung des Schlaftiefe-Index $S_F$ erforderlich ist.

**Patentansprüche**

1.    Vorrichtung zur Bestimmung der Schlaftiefe mit

-    einer Transformationseinrichtung (4), der ein Ausgangssignal (EEG(t)) einer EEG-Messapparatur (1) zugeführt wird und die das zugeführte EEG-Signal in den Frequenzbereich transformiert, und

-    einer Verarbeitungseinrichtung (5), der das in den Frequenzbereich transformierte EEG-Signal (EEG(f)) von der Transformationseinrichtung (4) zugeführt wird und die für den Frequenzbereich a von 4,75 bis 6,75 Hz, den Frequenzbereich b von 12,75 bis 18,5 Hz und den Frequenzbereich c von 18,75 bis 35,0 Hz oder zumindest zwei der Frequenzbereiche repräsentative Werte A, B und C ermittelt und daraus einen Schlaftiefe-Index $S_F$ bestimmt.

2.    Vorrichtung nach Anspruch 1,
dadurch **gekennzeichnet**, daß
die Verarbeitungseinrichtung (5) den Schlaftiefe-Index $S_F$ bestimmt als Quotienten aus der Summe der repräsentativen Werte A und B und dem repräsentativen Wert C

$$(S_F = \frac{A + B}{C}).$$

3. Vorrichtung nach Anspruch 1 oder 2,
dadurch **gekennzeichnet**, daß
die Verarbeitungseinrichtung (5) die repräsentativen Werte A, B und C durch Integration des EEG-Signals im Frequenzbereich ermittelt.

4. Vorrichtung nach einem der Ansprüche 1 oder 2,
dadurch **gekennzeichnet**, daß
die Verarbeitungseinrichtung (5) die repräsentativen Werte A, B und C durch Mittlung des EEG-Signals im Frequenzbereich ermittelt.

5. Vorrichtung nach einem der vorangegangenen Ansprüche,
dadurch **gekennzeichnet**, daß
die Transformationseinrichtung (4) das zugeführte EEG-Signal (EEG(t)) mittels einer schnellen Fourier-Transformation (FFT) in den Frequenzbereich transformiert.

# Fig. 1

# Fig. 2

**Europäisches Patentamt**

# EUROPÄISCHER RECHERCHENBERICHT

Nummer der Anmeldung

EP 98 12 2563

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 93 07804 A (ASPECT MEDICAL SYSTEMS INC) 29. April 1993 (1993-04-29) | 1,5 | A61B5/11 A61B5/048 |
| A | * Seite 4, Zeile 7 - Seite 5, Zeile 3 * | 2,4 | |
| | * Seite 7, Zeile 3 - Zeile 18 * | | |
| | * Seite 10, Zeile 1 - Zeile 24 * | | |
| | * Seite 14, Zeile 19 - Seite 16, Zeile 23 * | | |
| | * Seite 32, Zeile 26 - Seite 36, Zeile 25; Tabellen 1-4,11 * | | |
| | --- | | |
| A | US 5 699 808 A (JOHN ERWIN ROY) 23. Dezember 1997 (1997-12-23) | 1,5 | |
| | * Spalte 2, Zeile 35 - Zeile 65 * | | |
| | * Spalte 4, Zeile 5 - Zeile 50 * | | |
| | * Spalte 6, Zeile 17 - Zeile 26; Tabelle 1 * | | |
| | ----- | | |

**RECHERCHIERTE SACHGEBIETE (Int.Cl.7)**

A61B

Der vorliegende Recherchenbericht wurde für alle Patentansprüche erstellt

| Recherchenort | Abschlußdatum der Recherche | Prüfer |
|---|---|---|
| BERLIN | 11. November 1999 | Weihs, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**EP 0 974 304 A1**

## ANHANG ZUM EUROPÄISCHEN RECHERCHENBERICHT
## ÜBER DIE EUROPÄISCHE PATENTANMELDUNG NR.

EP 98 12 2563

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-11-1999

| Im Recherchenbericht angeführtes Patentdokument | Datum der Veröffentlichung | Mitglied(er) der Patentfamilie | Datum der Veröffentlichung |
|---|---|---|---|
| WO 9307804 A | 29-04-1993 | US 5320109 A | 14-06-1994 |
| | | AT 178198 T | 15-04-1999 |
| | | DE 69228823 D | 06-05-1999 |
| | | EP 0610365 A | 17-08-1994 |
| | | EP 0898234 A | 24-02-1999 |
| | | JP 7500983 T | 02-02-1995 |
| | | US 5458117 A | 17-10-1995 |
| US 5699808 A | 23-12-1997 | KEINE | |

Für nähere Einzelheiten zu diesem Anhang : siehe Amtsblatt des Europäischen Patentamts. Nr. 12/82